# EUROPEAN PATENT SPECIFICATION

(11) **EP 2 937 369 B1**
(45) Date of publication and mention of the grant of the patent: **11.11.2020**
(21) Application number: 13864081.8
(22) Date of filing: 19.12.2013
(51) Int. Cl.: C08G 18/80, C08G 18/18

(54) **QUATERNARY AMMONIUM SALT-CONTAINING CATALYST FOR DISSOCIATION OF BLOCKING AGENT, AND USE FOR SAID CATALYST**
QUATERNÄRES AMMONIUMSALZ MIT EINEM KATALYSATOR ZUR SPALTUNG EINES BLOCKIERUNGSMITTELS UND VERWENDUNG FÜR DEN KATALYSATOR
CATALYSEUR CONTENANT UN SEL D'AMMONIUM QUATERNAIRE POUR DISSOCIATION D'AGENT DE BLOCAGE ET UTILISATION DE CE CATALYSEUR

(30) Priority: 19.12.2012 JP 2012276856
(43) Date of publication of application: 28.10.2015
(73) Proprietor: Tosoh Corporation, Yamaguchi 746-8501 (JP)
(72) Inventor: FUJIWARA Hiroshi, Shunan-shi Yamaguchi 746-8501 (JP); KISO Hiroyuki, Shunan-shi Yamaguchi 746-8501 (JP)
(74) Representative: Vigand, Philippe
(86) International application number: PCT/JP2013/084061
(87) International publication number: WO 2014/098177

(56) References cited:
- GB-A- 1 506 196
- JP-A- H11 152 432
- JP-A- 2005 054 027
- JP-A- 2005 113 150
- JP-A- 2011 137 090
- JP-A- 2012 152 725
- JP-A- 2012 185 459
- JP-A- 2013 091 767
- US-A- 3 005 012

## Description

### Technical Field

The present invention relates to the use of a catalyst for dissociating a blocking agent of polyisocyanate (hereinafter sometimes referred to as "catalyst for dissociation of blocking agent") and to a thermosetting composition using a catalyst for dissociation of blocking agent.

Polyurethane resin coating materials have very excellent abrasion resistance, chemical resistance, and pollution resistance. General polyurethane resin coating materials are of a two-pack type composed of a polyol component and a polyisocyanate component, and after the respective components are separately stored, they are mixed and used at the time of coating. However, once the coating material is mixed, it cures for a short period of time, and thus, its pot life is short, so that there was involved a problem from the standpoint of workability at the time of coating. In addition, since the polyisocyanate easily reacts with water, it was impossible to use such a polyurethane resin coating material for a waterborne coating material as in an electrodeposition coating material. Thus, the conventional two-pack type polyurethane resin coating materials had a lot of restrictions on the occasion of using the same.

In order to improve the above-described problems, there is known a method of using a blocked isocyanate which is inactivated by allowing a polyisocyanate to react with an active hydrogen group-containing compound (blocking agent). This blocked isocyanate does not react with a polyol at room temperature, but when heated, the blocking agent is dissociated to regenerate an isocyanate group, whereby a crosslinking reaction with the polyol is proceeded. For this reason, the pot life is not restricted, and it is possible to mix the both in the coating material in advance to form a one-pack type, or to apply the blocked isocyanate to a waterborne coating material.

As a compound which is used as the blocking agent of polyisocyanate, there are, for example, known ε-caprolactam, methyl ethyl ketone oxime, phenol, and the like. However, as for a blocked isocyanate using such a compound, a high baking temperature as 140°C or higher is required for dissociating the blocking agent, and thus, there were involved such problems that such is disadvantageous from the standpoint of energy; and that the blocked isocyanate cannot be applied to plastic base materials having low heat resistance.

For this reason, there have hitherto been made attempts to make the baking temperature low by using a catalyst (catalyst for dissociation of blocking agent). As such a catalyst, organic tin compounds, such as dibutyltin dilaurate, etc., are known (for example, see Non-Patent Document 1); however, it may not be said that the use thereof is suitable in view of an issue of toxicity. In addition, a specified quaternary ammonium carboxylate is reported as the catalyst (for example, see Patent Document 1); however, it may not be said that a lowering of the dissociation temperature is sufficient. Therefore, a catalyst for dissociation of a blocking agent having a high dissociation effect at low temperatures is demanded.

### Background Art Document

### Patent Document

Patent Document 1: Japanese Patent No. 2732239

### Non-Patent Document

Non-Patent Document 1: Progress in Organic Coatings, Vol. 36, pp.148-172 (1999)

### Summary of Invention

### Problem that Invention is to Solve

In view of the foregoing background art, the present invention has been made, and its object is to provide the use of a catalyst for dissociation of a blocking agent having a high dissociation effect at low temperature and use thereof.

### Means for Solving Problem

In order to solve the above-described problem, the present inventors made extensive and intensive investigations. As a result, the present inventors have found that a catalyst containing a specified quaternary ammonium salt becomes a catalyst for dissociation of a blocking agent having a high dissociation effect at low temperatures; and that the solubility of the catalyst in a thermosetting composition increases, whereby a catalytic effect is enhanced, and have accomplished the present invention.

Specifically, the present invention is concerned with a thermosetting composition using a quaternary ammonium salt-containing catalyst for dissociation of a blocking agent, the use of a quaternary ammonium salt-containing catalyst for dissociation of a blocking agent, for dissociating a blocking agent from a blocked isocyanate, and a method for producing a thermosetting composition using a quaternary ammonium salt-containing catalyst for dissociation of a blocking agent.

The present invention is defined by independent claims 1, 4 and 6.

Different aspect of the catalyst for dissociation of a blocking agent used in the present invention are as shown in items [1] and [2] below.
[1] A catalyst for dissociation of a blocking agent, said catalyst for dissociation of a blocking agent comprising : a quaternary ammonium salt represented by the following general formula (1): 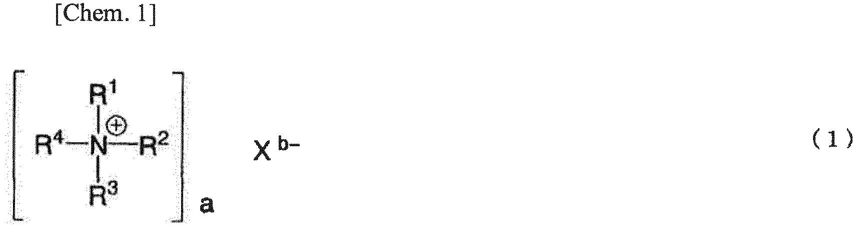 wherein each of R¹ to R³ represents an aliphatic hydrocarbon group having 1 to 8 carbon atoms or an aromatic hydrocarbon group having 6 to 14 carbon atoms; R⁴ represents an aliphatic hydrocarbon group having 9 to 20 carbon atoms; X represents at least one member selected from the group consisting of a phosphate group, a borate group, a hydrogencarbonate group, a monoalkylcarbonate group and a carbonate group; a is an integer in the range of 1 to 3; and b is an integer in the range of 1 to 3.
[2] The catalyst for dissociation of a blocking agent as described in [1] above,
   wherein the quaternary ammonium salt is at least one quaternary ammonium salt selected from the group consisting of trimethylmono-n-decylammonium orthophosphate, trimethylmono-n-decylammonium orthoborate, trimethylmono-n-decylammonium hydrogencarbonate, trimethylmono-n-decylammonium monomethylcarbonate, dimethylmonoethylmono-n-decylammonium monoethylcarbonate, trimethylmono-n-decylammonium carbonate, trimethylmono-n-dodecylammonium orthophosphate, trimethylmono-n-dodecylammonium orthoborate, trimethylmono-n-dodecylammonium hydrogencarbonate, trimethylmono-n-dodecylammonium monomethylcarbonate, dimethylmonoethylmono-n-dodecylammonium monoethylcarbonate, trimethylmono-n-dodecylammonium carbonate, trimethylmono-n-tetradecylammonium hydrogencarbonate, trimethylmono-n-tetradecylammonium monomethylcarbonate, trimethylmono-n-tetradecylammonium carbonate, trimethylmono-n-hexadecylammonium hydrogencarbonate, trimethylmono-n-hexadecylammonium monomethylcarbonate, trimethylmono-n-hexadecylammonium carbonate, trimethylmono-n-octadecylammonium hydrogencarbonate, trimethylmono-n-octadecylammonium monomethylcarbonate and trimethylmono-n-octadecylammonium carbonate.

### Effects of Invention

The catalyst for dissociation of a blocking agent used in the present invention shows that the catalytic activity for dissociating a blocking agent is better than that of known catalysts, such as organic tin compounds, etc., and therefore, it is extremely useful from the standpoint of industry.

In addition, the thermosetting composition of the present invention uses a catalyst for dissociation of a blocking agent having a high dissociation effect at low temperatures, and therefore, it is advantageous from the standpoint of energy and is also applicable to base materials having low heat resistance.

### Mode for Carrying Out Invention

The present invention is hereunder described in more detail.

The catalyst for dissociation of a blocking agent used in the present invention is characterized by containing a quaternary ammonium salt represented by the foregoing general formula (1). When the quaternary ammonium salt represented by the foregoing general formula (1) has an aliphatic hydrocarbon group having 9 to 20 carbon atoms, the solubility of the catalyst in a thermosetting composition increases, whereby the catalytic effect is enhanced.

In the foregoing general formula (1), in the case where R¹ to R³ are each an aliphatic hydrocarbon group, the aliphatic hydrocarbon group may be either linear or branched and may be either saturated or unsaturated. Examples of the aliphatic hydrocarbon group having 1 to 8 carbon atoms include a methyl group, an ethyl group, an n-propyl group, an isopropyl group, an n-butyl group, an isobutyl group, a sec-butyl group, a tert-butyl group, an n-pentyl group, an isopentyl group, a neopentyl group, a t-pentyl group, an n-hexyl group, a 1-methylpentyl group, a 4-methyl-2-pentyl group, a 3,3-dimethylbutyl group, a 2-ethylbutyl group, an n-heptyl group, a 1-methylhexyl group, an n-octyl group, a tert-octyl group, a 1-methylheptyl group, a 2-ethylhexyl group, a 2-propylpentyl group, and the like. In addition, in the foregoing general formula (1), in the case where R¹ to R³ are each an aromatic hydrocarbon group, the aromatic hydrocarbon group may be either monocyclic or polycyclic. Examples of the aromatic hydrocarbon group having 6 to 14 carbon atoms include a phenyl group, a (2-, 3-, or 4-)biphenylyl group, a (1- or 2-)naphthyl group, an acenaphthylen-(1-, 3-, 4-, or 5-)yl group, a fluoren-(1-, 2-, 3-, 4-, or 9-)yl group, a phenalene-(1- or 2-)yl group, a (1-, 2-, 3-, 4-, or 9-)phenanthryl group, and the like.

In the foregoing general formula (1), R⁴ represents an aliphatic hydrocarbon group having 9 to 20 carbon atoms, and the aliphatic hydrocarbon group may be either linear or branched and may be saturated or unsaturated. Examples of the aliphatic hydrocarbon group having 9 to 20 carbon atoms include an n-nonyl group, an n-decyl group, an n-undecyl group, an n-dodecyl group, an n-tridecyl group, an n-tetradecyl group, an n-pentadecyl group, an n-hexadecyl group, an n-heptadecyl group, an octadecyl group, an n-nonyldecyl group, an n-icosyl group, and the like. Since the quaternary ammonium salt used in the present invention contains a long-chain aliphatic hydrocarbon group as in R⁴ in a structure thereof, it has the advantage that the solubility in a thermosetting composition increases, whereby the catalytic effect is enhanced.

In addition, in the foregoing general formula (1), each of R¹ to R⁴ may have a substituent. Examples of such a substituent include the aliphatic hydrocarbon groups having 1 to 8 carbon atoms and the aromatic hydrocarbon groups having 6 to 14 carbon atoms as described above, and the like.

Furthermore, in the foregoing general formula (1), any two of R¹ to R⁴ may form an alicyclic ring or a heterocyclic ring via a carbon atom, an oxygen atom, or a nitrogen atom.

In the foregoing general formula (1), X^{b-} represents at least one member selected from the group consisting of a phosphate group, a borate group, a hydrogencarbonate group, a monoalkylcarbonate group and a carbonate group, and it is not particularly limited. Examples of the phosphate group include an orthophosphate group, a phosphite group, a hypophosphite group, a phosphinite group, a phosphenate group, a phosphenite group, a diphosphate group (pyrophosphate group), a triphosphate group, a metaphosphate group, and the like. Of these, an orthophosphate group and a phosphite group are especially preferred. In addition, examples of the borate group include an orthoborate group, a metaborate group, a perborate group, a hypoborate group, a boronate group (dihydroxyborane group), a borinate group (hydroxyborane group), and the like. Of these, an orthoborate group is especially preferred. In addition, examples of the monoalkylcarbonate group include monoalkylcarbonate groups having an alkyl group having 1 to 8 carbon atoms. Of these, a monomethylcarbonate group, a monoethylcarbonate group, a monopropylcarbonate group, and a monobutylcarbonate group are especially preferred.

In the present disclosure, a preparation method of the quaternary ammonium salt is not particularly limited. However, the quaternary ammonium salt may be prepared by, for example, a preparation method (1) of allowing a quaternary ammonium hydroxide to react with phosphoric acid, boric acid, or carbon dioxide; a preparation method (2) of allowing a quaternary ammonium monoalkylcarbonate obtained by a reaction between a tertiary amine and a carbonic acid diester to react with phosphoric acid, boric acid, carbon dioxide, or water; and the like.

Although a reaction condition of the above-described preparation method (1) is not particularly limited, it is preferred to perform the reaction in a solvent, such as water, ethanol, etc., at room temperature or under heating.

The quaternary ammonium hydroxide which is used in the above-described preparation method (1) is a compound represented by the following general formula (2).

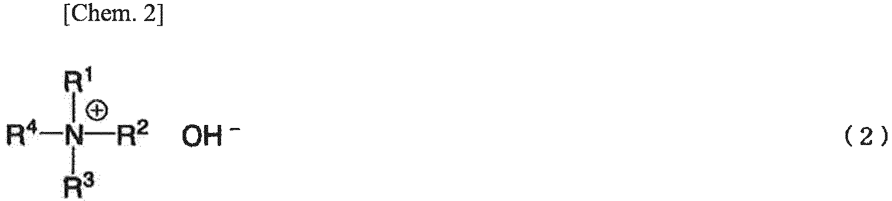

In the formula, each of R¹ to R³ represents an aliphatic hydrocarbon group having 1 to 8 carbon atoms or an aromatic hydrocarbon group having 6 to 14 carbon atoms; and R⁴ represents an aliphatic hydrocarbon group having 9 to 20 carbon atoms.

Specifically, examples of the quaternary ammonium hydroxide include trimethylmono-n-nonylammonium hydroxide, trimethylmono-n-decylammonium hydroxide, trimethylmono-n-undecylammonium hydroxide, trimethylmono-n-dodecylammonium hydroxide, trimethylmono-n-tridecylammonium hydroxide, trimethylmono-n-tetradecylammonium hydroxide, trimethylmono-n-pentadecylammonium hydroxide, trimethylmono-n-hexadecylammonium hydroxide, trimethylmono-n-heptadecylammonium hydroxide, trimethylmono-n-ooctadecylammonium hydroxide, trimethylmono-n-nonyldecylammonium hydroxide, trimethylmono-n-icosylammonium hydroxide, and the like.

A reaction condition of the above-described preparation method (2) is not particularly limited. However, it is preferred that the reaction between a tertiary amine and a carbonic acid diester is performed in a solvent, such as methanol, ethanol, etc., or in the absence of a solvent at room temperature or under heating. In addition, it is preferred that the reaction between a quaternary ammonium monoalkylcarbonate and phosphoric acid, boric acid, carbon dioxide, or water is performed in a solvent, such as methanol, ethanol, etc., or in the absence of a solvent at room temperature or under heating, and that a generated carbonic acid gas is properly removed from the reaction system, if necessary.

Although the tertiary amine which is used in the above-described preparation method (2) is not particularly limited, examples thereof include dimethylmono-n-nonylamine, dimethylmono-n-decylamine, dimethylmono-n-undecylamine, dimethylmono-n-dodecylamine, dimethylmono-n-tridecylamine, dimethylmono-n-tetradecylamine, dimethylmono-n-pentadecylamine, dimethylmono-n-hexadecylamine, dimethylmono-n-heptadecylamine, dimethylmono-n-octadecylamine, dimethylmono-n-nonyldecylamine, dimethylmono-n-icosylamine, and the like.

Although the carbonic acid diester which is used in the above-described preparation method (2) is not particularly limited, suitable examples thereof include dimethyl carbonate, diethyl carbonate, dipropyl carbonate, ethylmethyl carbonate, and the like.

Although the phosphoric acid which is used in the above-described preparation method (1) or (2) is not particularly limited, suitable examples thereof include orthophosphoric acid, phosphorous acid, hypophosphorous acid, phosphinous acid, phosphenic acid, phosphenous acid, diphosphoric acid (pyrophosphoric acid), triphosphoric acid, metaphosphoric acid, and the like.

Although the boric acid which is used in the above-described preparation method (1) or (2) is not particularly limited, suitable examples thereof include orthoboric acid, metaboric acid, perboric acid, hypoboric acid, boronic acid (dihydroxyborane), borinic acid (hydroxyborane), and the like.

The catalyst for dissociation of a blocking agent used in the present invention contains the quaternary ammonium salt represented by the foregoing general formula (1). Above all, it is preferred that the catalyst for dissociation of a blocking agent used in the present invention contains at least one quaternary ammonium salt selected from the group consisting of trimethylmono-n-decylammonium orthophosphate, trimethylmono-n-decylammonium orthoborate, trimethylmono-n-decylammonium hydrogencarbonate, trimethylmono-n-decylammonium monomethylcarbonate, dimethylmonoethylmono-n-decylammonium monoethylcarbonate, trimethylmono-n-decylammonium carbonate, trimethylmono-n-dodecylammonium orthophosphate, trimethylmono-n-dodecylammonium orthoborate, trimethylmono-n-dodecylammonium hydrogencarbonate, trimethylmono-n-dodecylammonium monomethylcarbonate, dimethylmonoethylmono-n-dodecylammonium monoethylcarbonate, trimethylmono-n-dodecylammonium carbonate, trimethylmono-n-tetradecylammonium hydrogencarbonate, trimethylmono-n-tetradecylammonium monomethylcarbonate, trimethylmono-n-tetradecylammonium carbonate, trimethylmono-n-hexadecylammonium hydrogencarbonate, trimethylmono-n-hexadecylammonium monomethylcarbonate, trimethylmono-n-hexadecylammonium carbonate, trimethylmono-n-octadecylammonium hydrogencarbonate, trimethylmono-n-octadecylammonium monomethylcarbonate, and trimethylmono-n-octadecylammonium carbonate, or the like.

The catalyst for dissociation of a blocking agent used in the present invention may contain a solvent, if necessary. Examples of the solvent include water, ethylene glycol, propylene glycol, 1,4-butanediol, toluene, benzene, methyl ethyl ketone, acetone, ethyl acetate, propylene glycol monomethyl ether acetate, diethylene glycol dimethyl ether, N-methylpyrrolidone, and the like. These solvents may be used solely, or may be used in combination of two or more kinds thereof. In the case where the thermosetting composition of the present invention is aqueous, a hydrophilic solvent is suitably used, whereas in the case where the thermosetting composition of the present invention is nonaqueous, a lipophilic solvent is suitably used.

Next, the thermosetting composition of the present invention is explained.

The thermosetting composition of the present invention contains the above-described catalyst for dissociation of a blocking agent, a blocked isocyanate, and a compound having an isocyanate reactive group.

In the thermosetting composition of the present invention, examples of the blocked isocyanate may include a nonaqueous blocked isocyanate, an aqueous blocked isocyanate, and the like.

Examples of the nonaqueous blocked isocyanate may include compounds obtained by blocking a known isocyanate compound or a prepolymer thereof by using a known blocking agent (for example, alcohols, such as methanol, ethanol, n-propanol, isopropanol, n-butanol, sec-butanol, tert-butanol, etc.; phenols, such as phenol, cresol, nitrophenol, chlorophenol, resorcinol, etc.; thiols, such as benzenethiol, etc.; caprolactams, such as ε-caprolactam, etc.; carbamates, such as ethyl carbamate, etc.; ketoenols, such as acetylacetone, etc.; ketooximes, such as methyl ethyl ketone oxime, etc.; amines, such as diisopropylamine, triazole, 3,5-dimethylpyrazole, etc.; sodium hydrogen sulfite; and the like).

Here, examples of the known isocyanate compound include aliphatic polyisocyanates, alicyclic polyisocyanates, aromatic polyisocyanates, aromatic aliphatic polyisocyanates, and the like.

Examples of the aliphatic polyisocyanate include 1,4-tetramethylene diisocyanate, 1,6-hexamethylene diisocyanate, 2,2,4-trimethylhexamethylene diisocyanate, 2,4,4-trimethylhexamethylene diisocyanate, lysine diisocyanate, dimer acid diisocyanate, and the like.

Examples of the alicyclic polyisocyanate include 1,3-bis(isocyanatomethyl)cyclohexane, 1,4-bis(isocyanatomethyl)cyclohexane, 3-isocyanatomethyl-3,3,5-trimethylcyclohexane (IPDI, isophorone diisocyanate), bis-(4-isocyanatocyclohexyl)methane (hydrogenated MDI), norbornane diisocyanate, and the like.

Examples of the aromatic polyisocyanate include 2,4'-diphenylmethane diisocyanate, 4,4'-diphenylmethane diisocyanate, crude MDI, 1,4-phenylene diisocyanate, 2,4-tolylene diisocyanate, 2,6-tolylene diisocyanate, 3,3'-dimethyl-4,4'-diisocyanatobiphenyl, 3,3'-dimethyl-4,4'-diisocyanatodiphenylmethane, 1,5-naphthylene diisocyanate, and the like.

Examples of the aromatic aliphatic polyisocyanate include 1,3-xylylene diisocyanate, 1,4-xylylene diisocyanate, α,α,α',α'-tetramethylxylylene diisocyanate, and the like.

In addition, examples of other isocyanate compounds than those described above may include isocyanate group-terminated compounds obtained by a reaction between an isocyanate compound and an active hydrogen group-containing compound, reaction products of these compounds (for example, adduct type polyisocyanates, isocyanate modification products by an allophanatization reaction, a carbodiimidation reaction, a uretodionization reaction, an isocyanuration reaction, a uretonimine reaction, a biuretization reaction, etc., and the like) or mixtures thereof, and the like.

Meanwhile, the aqueous blocked isocyanate may be obtained by, for example, allowing a polyisocyanate to react with a hydrophilic group having one or more active hydrogen groups capable of reacting with an isocyanate group and blocking the resultant by a known blocking agent. Examples of the hydrophilic group include ionic groups, such as a cation, an anion, etc., nonionic groups, and the like. Examples of the nonionic compound for introducing a nonionic group into the polyisocyanate include polyalkylene ether alcohols, polyoxyalkylene fatty acid esters, and the like.

In the thermosetting composition of the present invention, examples of the compound having an isocyanate reactive group include polyols. In the present invention, the polyol refers to a compound having two or more hydroxyl groups having reactivity with the isocyanate group, and specifically, examples thereof include nonaqueous polyols, aqueous polyols, and the like.

Examples of the nonaqueous polyol include acrylic polyols, polyester polyols, polyether polyols, epoxy polyols, and the like.

Examples of the acrylic polyol include copolymers of a polymerizable monomer having one or more active hydrogens in one molecule thereof and a monomer which is copolymerizable therewith.

Examples of the polymerizable monomer having one or more active hydrogens in one molecule thereof include acrylic acid hydroxy esters, such as 2-hydroxyethyl acrylate, 2-hydroxypropyl acrylate, 2-hydroxybutyl acrylate, etc.; methacrylic acid hydroxy esters, such as 2-hydroxyethyl methacrylate, 2-hydroxypropyl methacrylate, 2-hydroxybutyl methacrylate, etc.; acrylic acid monoesters or methacrylic acid monoesters of glycerin; acrylic acid monoesters or methacrylic acid monoesters of trimethylolpropane; monomers obtained by ring-opening polymerization of ε-caprolactone on such active hydrogen or hydrogens; and the like.

Examples of the monomer which is copolymerizable with the above-described polymerizable monomer include acrylic esters, such as methyl acrylate, ethyl acrylate, isopropyl acrylate, n-butyl acrylate, 2-ethylhexyl acrylate, etc.; methacrylic acid esters, such as methyl methacrylate, ethyl methacrylate, isopropyl methacrylate, n-butyl methacrylate, isobutyl methacrylate, n-hexyl methacrylate, cyclohexyl methacrylate, lauryl methacrylate, glycidyl methacrylate, etc.; unsaturated carboxylic acids, such as acrylic acid, methacrylic acid, maleic acid, itaconic acid, etc.; unsaturated amides, such as acrylamide, N-methylolacrylamide, diacetone acrylamide, etc.; styrene; vinyltoluene; vinyl acetate; acrylonitrile; and the like.

Examples of the polyester polyol include condensed polyester polyols, polycarbonate polyols, polylactone polyols, and the like.

Examples of the condensed polyester polyol include reaction products between a diol, such as ethylene glycol, propylene glycol, 1,3-propanediol, 1,4-butanediol, 1,5-pentanediol, 3-methyl-1,5-pentanediol, 1,6-hexanediol, neopentyl glycol, butylethylpropanediol, diethylene glycol, triethylene glycol, tetraethylene glycol, polyethylene glycol, dipropylene glycol, tripropylene glycol, etc.; and a dicarboxylic acid, such as succinic acid, adipic acid, azelaic acid, sebacic acid, dodecanedicarboxylic acid, maleic anhydride, fumaric acid, 1,3-cyclopentanedicarboxylic acid, 1,4-cyclohexanedicarboxylic acid, terephthalic acid, isophthalic acid, phthalic acid, naphthalenedicarboxylic acid, etc.

Specifically, there may be exemplified adipate-based condensed polyester diols, such as polyethylene adipate diol, polybutylene adipate diol, polyhexamethylene adipate diol, polyneopentylene adipate diol, polyethylenepropylene adipate diol, polyethylenebutylene adipate diol, polybutylenehexamethylene adipate diol, poly(polytetramethylene ether)adipate diol, etc.; azelate-based condensed polyester diols, such as polyethylene azelate diol, polybutylene azelate diol, etc.; and the like.

Examples of the polycarbonate polyol include reaction products between a diol, such as ethylene glycol, propylene glycol, 1,3-propanediol, 1,4-butanediol, 1,5-pentanediol, 3-methyl-1,5-pentanediol, 1,6-hexanediol, neopentyl glycol, butylethylpropane diol, diethylene glycol, triethylene glycol, tetraethylene glycol, polyethylene glycol, dipropylene glycol, tripropylene glycol, etc.; and a dialkyl carbonate, such as dimethyl carbonate, etc., and the like. Specifically, there are exemplified polytetramethylene carbonate diol, poly-3-methylpentamethylene carbonate diol, polyhexamethylene carbonate diol, and the like.

Examples of the polylactone polyol include ε-caprolactone, γ-butyrolactone, γ-valerolactone, and ring-opening polymers of a mixture of two or more kinds thereof, and the like. Specifically, there are exemplified polycaprolactone diol and the like.

Examples of the polyether polyol include reaction products obtained by addition polymerization of a monomer, such as ethylene oxide, propylene oxide, butylene oxide, styrene oxide, epichlorohydrin, tetrahydrofuran, cyclohexylene, etc., by using, as an initiator, a compound containing two or more active hydrogen atoms, such as ethylene glycol, propylene glycol, 1,3-butylene glycol, 1,4-butanediol, 1,6-hexanediol, diethylene glycol, neopentyl glycol, catechol, hydroquinone, bisphenol A, etc. In the case of a reaction product obtained by addition polymerization of two or more monomers, block addition, random addition, or a mixed system of the both may be adopted. Specifically, there are exemplified polyethylene glycol, polypropylene glycol, polytetramethylene ether glycol, and the like.

Examples of the epoxy polyol include epoxy polyols, such as novolak types, β-methylepichloro types, cyclic oxirane types, glycidyl ether types, glycol ether types, epoxy types of an aliphatic unsaturated compound, epoxidized fatty acid ester types, multivalent carboxylic acid ester types, aminoglycidyl types, halogenated types, resorcin types, etc.

In addition, as other nonaqueous polyols than those described above, for example, OH-terminated prepolymers produced by allowing such a polyol to react with an isocyanate compound may also be similarly used.

Meanwhile, examples of the aqueous polyol include compounds obtained by emulsifying, dispersing, or dissolving the above-described nonaqueous polyol in water. Examples of a method of emulsifying, dispersing, or dissolving the nonaqueous polyol in water include a method of introducing a carboxyl group, a sulfone group, or the like, followed by neutralization; and the like. Here, examples of a neutralizing agent include ammonia and water-soluble amino compounds, such as monoethanolamine, ethylamine, dimethylamine, diethylamine, triethylamine, propylamine, dipropylamine, isopropylamine, diisopropylamine, triethanolamine, butylamine, dibutylamine, 2-ethylhexylamine, methylethanolamine, dimethylethanolamine, diethylethanolamine, morpholine, etc. Of these, tertiary amines, such as triethylamine, dimethylethanolamine, etc., are suitably used.

In the thermosetting composition of the present invention, though a hydroxyl value of the polyol is not particularly limited, it is preferably in the range of 10 to 300 mgKOH/g, and more preferably in the range of 20 to 250 mgKOH/g. By controlling the hydroxyl value to 10 mgKOH/g or more, the resulting resin is enhanced in strength, whereas by controlling the hydroxyl value to 300 mgKOH/g or less, the resulting resin is enhanced in plasticity.

In the thermosetting composition of the present invention, though the polyol component is generally used as a composition containing a polyol (compound containing two or more hydroxyl groups having reactivity with the isocyanate group), a neutralizing agent, an antioxidant, and water, among these, the solid content means the polyol, the neutralizing agent, and the antioxidant.

The hydroxyl value of the polyol may be measured by the method prescribed in JIS K0070. That is, the hydroxyl value may be measured by subjecting a titration sample liquid prepared by dissolving a sample by the addition of acetic acid anhydride and pyridine and after allowing to stand for cooling, adding water and toluene thereto, to neutralization titration by an ethanol solution of potassium hydroxide. The hydroxyl value is expressed in terms of the number of mg of potassium hydroxide required for neutralizing acetic acid consumed for the purpose of acetylating the hydroxyl group contained in 1 g of the sample.

In the thermosetting composition of the present invention, an equivalent ratio of the hydroxyl group of the polyol and the isocyanate group ([hydroxyl group]/[isocyanate group]) is determined according to required physical properties of a coating film and is not particularly limited; however, it is generally in the range of 0.2 to 2.

In the thermosetting composition of the present invention, the use amount of the catalyst for dissociation of a blocking agent is in the range of generally 0.1 to 15% by weight, preferably 0.5 to 10% by weight, and more preferably 1 to 5% by weight in terms of the used amount of the above-described quaternary ammonium salt relative to the used amount of the blocked isocyanate ([used amount of the quaternary ammonium salt]/[used amount of the blocked isocyanate]). By controlling the used amount of the quaternary ammonium salt to 0.1% by weight or more relative to the used amount of the blocked isocyanate, sufficient low-temperature curability is obtained. On the other hand, even when the used amount of the quaternary ammonium salt exceeds 15% by weight relative to the used amount of the blocked isocyanate, a more enhancement of the low-temperature curability is not found, and such is economically disadvantageous.

In addition, in the thermosetting composition of the present invention, the used amount of the catalyst for dissociation of a blocking agent is in the range of generally 0.05 to 10% by weight, preferably 0.25 to 5% by weight, and more preferably 0.5 to 3% by weight in terms of the used amount of the above-described quaternary ammonium salt relative to the solid component ([used amount of the quaternary ammonium salt]/[the solid content]). In the present invention, the "solid content" expresses the component other than the solvent in the thermosetting composition, and for example, in the case of a nonaqueous thermosetting composition, it expresses a total sum of the component other than the solvent, such as butyl acetate, methyl ethyl ketone, etc., in the nonaqueous polyol and the component other than the solvent, such as methyl ethyl ketone, etc., in the nonaqueous blocked isocyanate; and in the case of an aqueous thermosetting composition, it expresses a total sum of the component other than the solvent, such as water, in the aqueous polyol and the component other than the solvent, such as water, etc., in the aqueous blocked isocyanate. By controlling the used amount of the quaternary ammonium salt to 0.05% by weight or more relative to the solid content, sufficient low-temperature curability is obtained. On the other hand, even when the used amount of the quaternary ammonium salt exceeds 10% by weight relative to the solid content, a more enhancement of the low-temperature curability is not found, and such is economically disadvantageous.

In the thermosetting composition of the present invention, an additive, a pigment, a solvent, and the like, which are commonly used in the subject technical field, may be used, if necessary.

Although the additive is not particularly limited, examples thereof include UV absorbers, such as hindered amine-based compounds, benzotriazole-based compounds, benzophenone-based compounds, etc.; coloring preventing agents, such as perchlorate-based compounds, hydroxylamine-based compounds, etc.; antioxidants, such as hindered phenol-based compounds, phosphorus-based compounds, sulfur-based compounds, hydrazine-based compounds, etc.; urethanization catalysts, such as tin-based compounds, zinc-based compounds, amine-based compounds, etc.; and besides, levelling agents, rheology controlling agents, pigment dispersants, and the like.

Although the pigment is not particularly limited, examples thereof include organic pigments, such as quinacridone-based compounds, azo-based compounds, phthalocyanine-based compounds, etc.; inorganic pigments, such as titanium oxide, barium sulfate, calcium carbonate, silica, etc.; and besides, carbon-based pigments, metallic foil-like pigments, rust preventive pigments, and the like.

Although the solvent is not particularly limited, examples thereof include hydrocarbons, such as benzene, toluene, xylene, cyclohexane, mineral spirits, naphtha, etc.; ketones, such as acetone, methyl ethyl ketone, methyl isobutyl ketone, etc.; and esters, such as ethyl acetate, n-butyl acetate, cellosolve acetate, etc. These solvents may be used solely, or may be used in combination of two or more kinds thereof.

The thermosetting composition of the present invention may be used as a top or intermediate coating material of automobile, an anti-chipping coating material, an electrodeposition coating material, a coating material for automobile parts, a coating material for automobile repair, a coating material for precoat metal or rust preventive steel sheet of metal products, such as consumer electrical appliances, office machines, etc., or building materials, a coating material for plastics, an adhesive, an adhesion imparting agent, a sealing agent, and the like.

### Examples

The present invention is more specifically explained by reference to the following Examples, but it should be construed that the present invention is not limited by these Examples at all.

It is to be noted that in the following Examples, baking of the thermosetting composition and measurement of the solvent resistance were carried out as follows.

### <Baking of thermosetting composition>

The thermosetting composition was coated on an aluminum sheet (A1050, manufactured by Paltec Test Panels Co., Ltd.), preliminarily dried in an oven at 50°C for 30 minutes, and then put into an oven at a prescribed temperature, followed by baking for 30 minutes.

### <Measurement of solvent resistance>

The above-described baked coating film was cooled to room temperature and then rubbed by absorbent cotton soaked with methyl ethyl ketone, and the number of reciprocations until the coating film surface was scratched was measured, thereby evaluating the solvent resistance.

### Production Example 1 (Preparation of nonaqueous blocked isocyanate)

In a four-necked flask equipped with a nitrogen inlet tube, a stirring blade was installed, the interior of the vessel was rendered in a nitrogen atmosphere, and then, 50.2 g of CORONATE HX (manufactured by Nippon Polyurethane Industry Co., Ltd., hexamethylene diisocyanate trimer, NCO: 21.3% by weight) and 114 g of dehydrated methyl ethyl ketone were charged into the vessel, followed by stirring at 40°C for 5 minutes. Thereafter, a dropping funnel was installed in the vessel, and 22.2 g of methyl ethyl ketone oxime was added dropwise into the vessel over 1 hour while keeping at 40°C. Then, a reflux condenser was installed in the vessel, the contents were allowed to react at 70°C for 1 hour, and after the isocyanate was not detected, the resultant was cooled to room temperature to stop the reaction, and thereby a nonaqueous blocked isocyanate was obtained. The obtained nonaqueous blocked isocyanate had a solid content concentration of 40.0% by weight and an effective NCO of 1.36 mmol/g.

Here, the effective NCO means an amount of the isocyanate group (NCO) which becomes possible to undergo the reaction, when the blocked isocyanate is heated and the blocking agent is dissociated. Then, "the effective NCO is 1.36 mmol/g" means that 1.36 mmol of the isocyanate group is latently contained in 1 g of the blocked isocyanate (it is regenerated by the blocking agent being dissociated).

### Production Example 2 (Preparation of aqueous blocked isocyanate)

In a four-necked flask equipped with a nitrogen inlet tube, a stirring blade and a reflux condenser were installed, and the interior of the vessel was rendered in a nitrogen atmosphere. Subsequently, 49.0 g of CORONATE HX (manufactured by Nippon Polyurethane Industry Co., Ltd., hexamethylene diisocyanate trimer, NCO: 21.3% by weight) and 13.7 g of polyethylene glycol monomethyl ether (manufactured by Aldrich, average molecular weight: 550) were charged into the vessel, and the contents were allowed to react at 80°C for 9 hours. Thereafter, 18.6 g of methyl ethyl ketone oxime and 20.0 g of methyl ethyl ketone were added into the vessel, the contents were allowed to react at 80°C for 3 hours, and after the isocyanate was not detected, the resultant was cooled to room temperature to stop the reaction.

To 100 g of the obtained composition, 150 g of water was gradually added while stirring, thereby emulsifying and dispersing the composition in water. The remaining methyl ethyl ketone was removed from the obtained emulsion dispersion liquid by an evaporator. The obtained aqueous blocked isocyanate was a stable dispersion liquid having a solid content concentration of 39.0% by weight and an effective NCO of 1.19 mmol/g.

### Production Example 3 (Preparation of quaternary ammonium salt)

In a 200-mL autoclave, 20.0 g of dimethylmono-n-decylamine, 14.6 g of dimethyl carbonate, and 34.6 g of methanol were charged, and the contents were allowed to react with stirring at 110°C for 12 hours. The resultant was recovered in a one-necked eggplant type flask (recovery amount: 65.7 g), and 42.4 g of ethylene glycol was added thereto. Thereafter, the interior of the vessel was controlled to 30°C and evacuated to remove the unreacted dimethyl carbonate and methanol, and thereby 70.6 g of a 40.0% by weight ethylene glycol solution of trimethylmono-n-decylammonium monomethylcarbonate was obtained.

### Production Example 4 (Preparation of quaternary ammonium salt)

In a Griffin beaker, 30.0 g of the 40.0% by weight ethylene glycol solution of trimethylmono-n-decylammonium monomethylcarbonate obtained in Production Example 3 and 1.67 g of an 85% aqueous solution of orthophosphoric acid (manufactured by Kishida Chemical Co., Ltd.), and the contents were allowed to react with stirring at 25°C for 1 hour, and thereby 29.8 g of a 33.9% by weight ethylene glycol solution of trimethylmono-n-decylammonium orthophosphate was obtained.

### Production Example 5 (Preparation of quaternary ammonium salt)

In a Griffin beaker, 30.0 g of the 40.0% by weight ethylene glycol solution of trimethylmono-n-decylammonium monomethylcarbonate obtained in Production Example 3 and 2.69 g of orthoboric acid (manufactured by Kishida Chemical Co., Ltd.), and the contents were allowed to react with stirring at 25°C for 1 hour, and thereby 30.8 g of a 37.1% by weight ethylene glycol solution of trimethylmono-n-decylammonium orthoborate was obtained.

### Production Example 6 (Preparation of quaternary ammonium salt)

In a Griffin beaker, 30.0 g of the 40.0% by weight ethylene glycol solution of trimethylmono-n-decylammonium monomethylcarbonate obtained in Production Example 3 was charged and allowed to react with stirring at 25°C while bubbling carbon dioxide by using a carbon dioxide cylinder. The reaction was continued for 5 hours, and thereby 29.4 g of a 38.7% by weight ethylene glycol solution of trimethylmono-n-decylammonium hydrogencarbonate was obtained.

### Production Example 7 (Preparation of quaternary ammonium salt)

In a 200-mL autoclave, 20.0 g of dimethylmono-n-decylamine, 19.1 g of diethyl carbonate, and 34.6 g of methanol were charged, and the contents were allowed to react with stirring at 150°C for 12 hours. The resultant was recovered in a one-necked eggplant type flask (recovery amount: 70.0 g), and 46.7 g of ethylene glycol was added thereto. Thereafter, the interior of the vessel was controlled to 30°C and evacuated to remove the unreacted diethyl carbonate and methanol, and thereby 77.8 g of a 40.0% by weight ethylene glycol solution of dimethymonoethylmono-n-decylammonium monoethylcarbonate was obtained.

### Production Example 8 (Preparation of quaternary ammonium salt)

In a 200-mL autoclave, 20.0 g of dimethylmono-n-decylamine and 48.6 g of dimethyl carbonate were charged, and the contents were allowed to react with stirring at 110°C for 12 hours. The resultant was recovered in a one-necked eggplant type flask (recovery amount: 61.7 g), and 33.4 g of ethylene glycol was added thereto. Thereafter, the interior of the vessel was controlled to 30°C and evacuated to remove the unreacted dimethyl carbonate, and thereby 55.9 g of a 40.0% by weight ethylene glycol solution of trimethylmono-n-decylammonium carbonate was obtained.

### Production Example 9 (Preparation of quaternary ammonium salt)

In a 200-mL autoclave, 20.0 g of dimethylmono-n-dodecylamine, 12.7 g of dimethyl carbonate, and 30.0 g of methanol were charged, and the contents were allowed to react with stirring at 120°C for 12 hours. The resultant was recovered in a one-necked eggplant type flask (recovery amount: 59.6 g), and 40.5 g of ethylene glycol was added thereto. Thereafter, the interior of the vessel was controlled to 30°C and evacuated to remove the unreacted dimethyl carbonate and methanol, and thereby 67.6 g of a 40.0% by weight ethylene glycol solution of trimethylmono-n-dodecylammonium monomethylcarbonate was obtained.

### Production Example 10 (Preparation of quaternary ammonium salt)

In a Griffin beaker, 30.0 g of the 40.0% by weight ethylene glycol solution of trimethylmono-n-dodecylammonium monomethylcarbonate obtained in Production Example 9 and 1.52 g of an 85% aqueous solution of orthophosphoric acid (manufactured by Kishida Chemical Co., Ltd.), and the contents were allowed to react with stirring at 25°C for 1 hour, and thereby 29.8 g of a 34.5% by weight ethylene glycol solution of trimethylmono-n-dodecylammonium orthophosphate was obtained.

### Production Example 11 (Preparation of quaternary ammonium salt)

In a Griffin beaker, 30.0 g of the 40.0% by weight ethylene glycol solution of trimethylmono-n-dodecylammonium monomethylcarbonate obtained in Production Example 9 and 2.44 g of orthoboric acid (manufactured by Kishida Chemical Co., Ltd.), and the contents were allowed to react with stirring at 25°C for 1 hour, and thereby 30.7 g of a 37.4% by weight ethylene glycol solution of trimethylmono-n-dodecylammonium orthoborate was obtained.

### Production Example 12 (Preparation of quaternary ammonium salt)

In a Griffin beaker, 30.0 g of the 40.0% by weight ethylene glycol solution of trimethylmono-n-dodecylammonium monomethylcarbonate obtained in Production Example 9 was charged and allowed to react with stirring at 25°C while bubbling carbon dioxide by using a carbon dioxide cylinder. The reaction was continued for 5 hours, and thereby 29.4 g of a 38.9% by weight ethylene glycol solution of trimethylmono-n-dodecylammonium hydrogencarbonate was obtained.

### Production Example 13 (Preparation of quaternary ammonium salt)

In a 200-mL autoclave, 20.0 g of dimethylmono-n-dodecylamine, 16.6 g of diethyl carbonate, and 30.0 g of methanol were charged, and the contents were allowed to react with stirring at 150°C for 12 hours. The resultant was recovered in a one-necked eggplant type flask (recovery amount: 63.3 g), and 44.3 g of ethylene glycol was added thereto. Thereafter, the interior of the vessel was controlled to 30°C and evacuated to remove the unreacted diethyl carbonate and methanol, and thereby 73.8 g of a 40.0% by weight ethylene glycol solution of dimethylmonoethylmono-n-dodecylammonium monoethylcarbonate was obtained.

### Production Example 14 (Preparation of quaternary ammonium salt)

In a 200-mL autoclave, 20.0 g of dimethylmono-n-dodecylamine and 42.2 g of dimethyl carbonate were charged, and the contents were allowed to react with stirring at 120°C for 12 hours. The resultant was recovered in a one-necked eggplant type flask (recovery amount: 56.0 g), and 32.7 g of ethylene glycol was added thereto. Thereafter, the interior of the vessel was controlled to 30°C and evacuated to remove the unreacted dimethyl carbonate, and thereby 54.5 g of a 40.0% by weight ethylene glycol solution of trimethylmono-n-dodecylammonium carbonate was obtained.

### Production Example 15 (Preparation of quaternary ammonium salt)

In a 200-mL autoclave, 20.0 g of dimethylmono-n-tetradecylamine, 11.2 g of dimethyl carbonate, and 26.5 g of methanol were charged, and the contents were allowed to react with stirring at 130°C for 12 hours. The resultant was recovered in a one-necked eggplant type flask (recovery amount: 54.8 g), and 39.1 g of ethylene glycol was added thereto. Thereafter, the interior of the vessel was controlled to 30°C and evacuated to remove the unreacted dimethyl carbonate and methanol, and thereby 65.2 g of a 40.0% by weight ethylene glycol solution of trimethylmono-n-tetradecylammonium monomethylcarbonate was obtained.

### Production Example 16 (Preparation of quaternary ammonium salt)

In a Griffin beaker, 30.0 g of the 40.0% by weight ethylene glycol solution of trimethylmono-n-tetradecylammonium monomethylcarbonate obtained in Production Example 15 was charged and allowed to react with stirring at 25°C while bubbling carbon dioxide by using a carbon dioxide cylinder. The reaction was continued for 5 hours, and thereby 29.5 g of a 39.0% by weight ethylene glycol solution of trimethylmono-n-tetradecylammonium hydrogencarbonate was obtained.

### Production Example 17 (Preparation of quaternary ammonium salt)

In a 200-mL autoclave, 20.0 g of dimethylmono-n-tetradecylamine and 37.3 g of dimethyl carbonate were charged, and the contents were allowed to react with stirring at 130°C for 12 hours. The resultant was recovered in a one-necked eggplant type flask (recovery amount: 51.6 g), and 32.0 g of ethylene glycol was added thereto. Thereafter, the interior of the vessel was controlled to 30°C and evacuated to remove the unreacted dimethyl carbonate, and thereby 53.4 g of a 40.0% by weight ethylene glycol solution of trimethylmono-n-tetradecylammonium carbonate was obtained.

### Production Example 18 (Preparation of quaternary ammonium salt)

In a 200-mL autoclave, 20.0 g of dimethylmono-n-hexadecylamine, 10.0 g of dimethyl carbonate, and 23.8 g of methanol were charged, and the contents were allowed to react with stirring at 140°C for 12 hours. The resultant was recovered in a one-necked eggplant type flask (recovery amount: 51.1 g), and 38.0 g of ethylene glycol was added thereto. Thereafter, the interior of the vessel was controlled to 30°C and evacuated to remove the unreacted dimethyl carbonate and methanol, and thereby 63.4 g of a 40.0% by weight ethylene glycol solution of trimethylmono-n-hexadecylammonium monomethylcarbonate was obtained.

### Production Example 19 (Preparation of quaternary ammonium salt)

In a Griffin beaker, 30.0 g of the 40.0% by weight ethylene glycol solution of trimethylmono-n-hexadecylammonium monomethylcarbonate obtained in Production Example 18 was charged and allowed to react with stirring at 25°C while bubbling carbon dioxide by using a carbon dioxide cylinder. The reaction was continued for 5 hours, and thereby 29.5 g of a 39.1% by weight ethylene glycol solution of trimethylmono-n-hexadecylammonium hydrogencarbonate was obtained.

### Production Example 20 (Preparation of quaternary ammonium salt)

In a 200-mL autoclave, 20.0 g of dimethylmono-n-hexadecylamine and 33.4 g of dimethyl carbonate were charged, and the contents were allowed to react with stirring at 140°C for 12 hours. The resultant was recovered in a one-necked eggplant type flask (recovery amount: 48.1 g), and 31.5 g of ethylene glycol was added thereto. Thereafter, the interior of the vessel was controlled to 30°C and evacuated to remove the unreacted dimethyl carbonate, and thereby 52.5 g of a 40.0% by weight ethylene glycol solution of trimethylmono-n-hexadecylammonium carbonate was obtained.

### Production Example 21 (Preparation of quaternary ammonium salt)

In a 200-mL autoclave, 20.0 g of dimethylmono-n-octadecylamine, 9.08 g of dimethyl carbonate, and 21.5 g of methanol were charged, and the contents were allowed to react with stirring at 150°C for 12 hours. The resultant was recovered in a one-necked eggplant type flask (recovery amount: 48.1 g), and 37.1 g of ethylene glycol was added thereto. Thereafter, the interior of the vessel was controlled to 30°C and evacuated to remove the unreacted dimethyl carbonate and methanol, and thereby 61.9 g of a 40.0% by weight ethylene glycol solution of trimethylmono-n-octadecylammonium monomethylcarbonate was obtained.

### Production Example 22 (Preparation of quaternary ammonium salt)

In a Griffin beaker, 30.0 g of the 40.0% by weight ethylene glycol solution of trimethylmono-n-octadecylammonium monomethylcarbonate obtained in Production Example 21 was charged and allowed to react with stirring at 25°C while bubbling carbon dioxide by using a carbon dioxide cylinder. The reaction was continued for 5 hours, and thereby 29.6 g of a 39.1% by weight ethylene glycol solution of trimethylmono-n-octadecylammonium hydrogencarbonate was obtained.

### Production Example 23 (Preparation of quaternary ammonium salt)

In a 200-mL autoclave, 20.0 g of dimethylmono-n-octadecylamine and 30.3 g of dimethyl carbonate were charged, and the contents were allowed to react with stirring at 150°C for 12 hours. The resultant was recovered in a one-necked eggplant type flask (recovery amount: 45.2 g), and 31.1 g of ethylene glycol was added thereto. Thereafter, the interior of the vessel was controlled to 30°C and evacuated to remove the unreacted dimethyl carbonate, and thereby 51.8 g of a 40.0% by weight ethylene glycol solution of trimethylmono-n-octadecylammonium carbonate was obtained.

### Example 1 (Evaluation of catalytic activity of quaternary ammonium salt in nonaqueous thermosetting composition)

A nonaqueous acrylic polyol (ACRYDIC A-801, manufactured by DIC Corporation, solid content concentration: 50.2% by weight, hydroxyl value relative to the solid content: 102 mgKOH/g) and the nonaqueous blocked isocyanate obtained in Production Example 1 were mixed in a formulation shown in Table 1, and thereafter, the 40.0% by weight ethylene glycol solution of trimethylmono-n-decylammonium monomethylcarbonate obtained in Production Example 3 was added thereto while stirring, and thereby a nonaqueous thermosetting composition containing trimethylmono-n-decylammonium monomethylcarbonate was obtained.

The obtained nonaqueous thermosetting composition was baked at 80°C, 90°C, 100°C, 110°C, and 120°C, respectively and then measured for the solvent resistance.

### Examples 2 to 21

Nonaqueous thermosetting compositions containing a quaternary ammonium salt were obtained in formations shown in Tables 1 to 4 by the same method as that in Example 1.

Each of the obtained nonaqueous thermosetting compositions was baked at 80°C, 90°C, 100°C, 110°C, and 120°C, respectively and then measured for the solvent resistance.

The foregoing results are collectively shown in Tables 1 to 4.

**Table 1**

| | Example 1 | Example 2 | Example 3 | Example 4 | Example 5 | Example 6 |
|---|---|---|---|---|---|---|
| Nonaqueous acrylic polyol ⁽¹⁾ | 10.0 g | 10.0 g | 10.0 g | 10.0 g | 10.0 g | 10.0 g |
| Nonaqueous blocked isocyanate ⁽²⁾ | 6.78 g | 6.78 g | 6.78 g | 6.78 g | 6.78 g | 6.78 g |
| Catalyst solution | 40.0% by weight ethylene glycol solution of trimethylmono-n-decylammonium monomethylcarbonate obtained in Production Example 3 | 33.9% by weight ethylene glycol solution of trimethylmono-n-decylammonium orthophosphate obtained in Production Example 4 | 37.1% by weight ethylene glycol solution of trimethylmono- n-decylammonium orthoborate obtained in Production Example 5 | 38.7% by weight ethylene glycol solution of trimethylmono- n-decylammonium hydrogencarbonate obtained in Production Example 6 | 40.0% by weight ethylene glycol solution of dimethylmonoethylmono-n-decylammonium monoethylcarbonate obtained in Production Example 7 | 40.0% by weight ethylene glycol solution of trimethylmono-n-dec ylammonium carbonate obtained in Production Example 8 |
| | 0.191 g | 0.225 g | 0.206 g | 0.197 g | 0.191 g | 0.191 g |
| Proportion of catalyst to solid content | 1% by weight | 1% by weight | 1% by weight | 1% by weight | 1% by weight | 1% by weight |
| Solvent resistance (rubbing number) | | | | | | |
| 80°C ⁽³⁾ | 40 | 30 | <5 | 35 | 25 | 35 |
| 90°C ⁽³⁾ | >100 | >100 | 20 | >100 | >100 | >100 |
| 100°C ⁽³⁾ | >100 | >100 | >100 | >100 | >100 | >100 |
| 110°C ⁽³⁾ | >100 | >100 | >100 | >100 | >100 | >100 |
| 120°C ⁽³⁾ | >100 | >100 | >100 | >100 | >100 | >100 |

| | | | | | | |
|---|---|---|---|---|---|---|
| (1) ACRYDIC A-801, manufactured by DIC Corporation (2) Nonaqueous blocked isocyanate obtained in Production Example 1 (3) Baking temperature | | | | | | |

**Table 2**

| | Example 7 | Example 8 | Example 9 | Example 10 | Example 11 | Example 12 |
|---|---|---|---|---|---|---|
| Nonaqueous acrylic polyol ⁽¹⁾ | 10.0 g | 10.0 g | 10.0 g | 10.0 g | 10.0 g | 10.0 g |
| Nonaqueous blocked isocyanate ⁽²⁾ | 6.78 g | 6.78 g | 6.78 g | 6.78 g | 6.78 g | 6.78 g |
| Catalyst solution | 40.0% by weight ethylene glycol solution of trimethylmono-n-dodecy lammonium monomethylcarbonate obtained in Production Example 9 | 34.5% by weight ethylene glycol solution of trimethylmono-n-dodecy lammonium orthophosphate obtained in Production Example 10 | 37.4% by weight ethylene glycol solution of trimethylmono- n-dodecy lammonium orthoborate obtained in Production Example 11 | 38.9% by weight ethylene glycol solution of trimethylmono- n-dodecy lammonium hydrogencarbonate obtained in Production Example 12 | 40.0% by weight ethylene glycol solution of dimethylmonoethylmono-n-dodecylammonium monoethylcarbonate obtained in Production Example 13 | 40.0% by weight ethylene glycol solution of trimethylmono- n-dodecy lammonium carbonate obtained in Production Example 14 |
| | 0.191 g | 0.221 g | 0.204 g | 0.196 g | 0.191 g | 0.191 g |
| Proportion of catalyst to solid content | 1% by weight | 1% by weight | 1% by weight | 1% by weight | 1% by weight | 1% by weight |
| Solvent resistance (rubbing number) | | | | | | |
| 80°C ⁽³⁾ | 40 | 30 | <5 | 40 | 25 | 35 |
| 90°C ⁽³⁾ | >100 | >100 | 25 | >100 | >100 | >100 |
| 100°C ⁽³⁾ | >100 | >100 | >100 | >100 | >100 | >100 |
| 110°C ⁽³⁾ | >100 | >100 | >100 | >100 | >100 | >100 |
| 120°C ⁽³⁾ | >100 | >100 | >100 | >100 | >100 | >100 |

| | | | | | | |
|---|---|---|---|---|---|---|
| (1) ACRYDIC A-801, manufactured by DIC Corporation (2) Nonaqueous blocked isocyanate obtained in Production Example 1 (3) Baking temperature | | | | | | |

**Table 3**

| | Example 13 | Example 14 | Example 15 | Example 16 | Example 17 | 18 |
|---|---|---|---|---|---|---|
| Nonaqueous acrylic polyol ⁽¹⁾ | 10.0 g | 10.0 g | 10.0 g | 10.0 g | 10.0 g | 10.0 g |
| Nonaqueous blocked isocyanate ⁽²⁾ | 6.78 g | 6.78 g | 6.78 g | 6.78 g | 6.78 g | 6.78 g |
| Catalyst solution | 40.0% by weight ethylene glycol solution of trimethylmono-n-tetradecylammonium monomethylcarbonate obtained in Production Example 15 0.191 g | 39.0% by weight ethylene glycol solution of trimethylmono- n-tetradecylammonium hydrogencarbonate obtained in Production Example 16 0.196 g | 40.0% by weight ethylene glycol solution of trimethylmono- n-tetradecylammonium carbonate obtained in Production Example 17 0.191 g | 40.0% by weight ethylene glycol solution of trimethylmono-n-hexadecylammonium monomethylcarbonate obtained in Production Example 18 0.191 g | 39.1% by weight ethylene glycol solution of trimethylmono-n-hexadecylammonium hydrogencarbonate obtained in Production Example 19 0.195 g | 40.0% by weight ethylene glycol solution of trimethylmono-n-hexadecylammonium carbonate obtained in Production Example 20 0.191 g |
| Proportion of catalyst to solid content | 1% by weight | 1% by weight | 1% by weight | 1% by weight | 1% by weight | 1% by weight |
| Solvent resistance (rubbing number) | | | | | | |
| 80°C ⁽³⁾ | 40 | 30 | 35 | 35 | 30 | 35 |
| 90°C ⁽³⁾ | >100 | >100 | >100 | >100 | >100 | >100 |
| 100°C ⁽³⁾ | >100 | >100 | >100 | >100 | >100 | >100 |
| 110°C ⁽³⁾ | >100 | >100 | >100 | >100 | >100 | >100 |
| 120°C ⁽³⁾ | >100 | >100 | >100 | >100 | >100 | >100 |

| | | | | | | |
|---|---|---|---|---|---|---|
| (1) ACRYDIC A-801, manufactured by DIC Corporation (2) Nonaqueous blocked isocyanate obtained in Production Example 1 (3) Baking temperature | | | | | | |

**Table 4**

| | Example 19 | Example 20 | Example 21 |
|---|---|---|---|
| Nonaqueous acrylic polyol ⁽¹⁾ | 10.0 g | 10.0 g | 10.0 g |
| Nonaqueous blocked isocyanate ⁽²⁾ | 6.78 g | 6.78 g | 6.78 g |
| Catalyst solution | 40.0% by weight ethylene glycol solution of trimethylmono-n-octadecylammonium monomethylcarbonate obtained in Production Example 21 | 39.1% by weight ethylene glycol solution of trimethylmono-n-octadecylammonium hydrogencarbonate obtained in Production Example 22 | 40.0% by weight ethylene glycol solution of trimethylmono-n-octadodecyl-ammonium carbonate obtained in Production Example 23 |
| | 0.191 g | 0.195 g | 0.191 g |
| Proportion of catalyst to solid content | 1% by weight | 1% by weight | 1% by weight |
| Solvent resistance (rubbing number) | | | |
| 80°C ⁽³⁾ | 35 | 30 | 30 |
| 90°C ⁽³⁾ | >100 | >100 | >100 |
| 100°C ⁽³⁾ | >100 | >100 | >100 |
| 110°C ⁽³⁾ | >100 | >100 | >100 |
| 120°C ⁽³⁾ | >100 | >100 | >100 |

| | | | |
|---|---|---|---|
| (1) ACRYDIC A-801, manufactured by DIC Corporation (2) Nonaqueous blocked isocyanate obtained in Production Example 1 (3) Baking temperature | | | |

### Comparative Example 1 (Evaluation of curability in non-addition of catalyst in nonaqueous thermosetting composition)

A nonaqueous acrylic polyol (ACRYDIC A-801, manufactured by DIC Corporation, solid content concentration: 50.2% by weight, hydroxyl value relative to the solid content: 102 mgKOH/g) and the nonaqueous blocked isocyanate obtained in Production Example 1 were mixed in a formulation shown in Table 5, and thereby a catalyst-free nonaqueous thermosetting composition was obtained.

**Table 5**

| | Comparative Example 1 | Comparative Example 2 | Comparative Example 3 |
|---|---|---|---|
| Nonaqueous acrylic polyol ⁽¹⁾ | 10.0 g | 10.0 g | 10.0 g |
| Nonaqueous blocked isocyanate ⁽²⁾ | 6.78 g | 6.78 g | 6.78 g |
| Catalyst solution | No | 8% by weight propylene glycol monomethyl ether acetate solution of dibutyltin dilaurate 0.954 g | 40.0% by weight aqueous solution of triethylmonomethylammonium 2-ethylhexanoate 0.191 g |
| Proportion of catalyst to solid content | 0% by weight | 1% by weight | 1% by weight |
| Solvent resistance (rubbing number) | | | |
| 80°C ⁽³⁾ | <5 | <5 | <5 |
| 90°C ⁽³⁾ | <5 | <5 | <5 |
| 100°C ⁽³⁾ | <5 | <5 | <5 |
| 110°C ⁽³⁾ | <5 | <5 | 30 |
| 120°C ⁽³⁾ | <5 | 25 | >100 |

| | | | |
|---|---|---|---|
| (1) ACRYDIC A-801, manufactured by DIC Corporation (2) Nonaqueous blocked isocyanate obtained in Production Example 1 (3) Baking temperature | | | |

The obtained nonaqueous thermosetting composition was baked at 80°C, 90°C, 100°C, 110°C, and 120°C, respectively and then measured for the solvent resistance. The results are shown in Table 5.

As is clear from Tables 1 to 5, in Comparative Example 1, the rubbing number is less than 5 times at 120°C, and the coating film is not cured. On the other hand, in view of the fact that the rubbing number reaches 100 times at 90°C in Examples 1, 2, 4 to 8, and 10 to 21 and at 100°C in Examples 3 and 9, respectively, and the coating films are cured, it is understood that the dissociation temperature of the blocking agent was lowered by the addition of the quaternary ammonium salt.

### Comparative Example 2 (Evaluation of curability of known catalyst in nonaqueous thermosetting composition)

A nonaqueous acrylic polyol (ACRYDIC A-801, manufactured by DIC Corporation, solid content concentration: 50.2% by weight, hydroxyl value relative to the solid content: 102 mgKOH/g) and the nonaqueous blocked isocyanate obtained in Production Example 1 were mixed in a formulation shown in Table 5, and thereafter, an 8% by weight propylene glycol monomethyl ether acetate of dibutyltin dilaurate was added thereto while stirring, and thereby a known catalyst-containing nonaqueous thermosetting composition was obtained.

The obtained nonaqueous thermosetting composition was baked at 80°C, 90°C, 100°C, 110°C, and 120°C, respectively and then measured for the solvent resistance. The results are collectively shown in Table 5.

As is clear from Tables 1 to 5, in Comparative Example 2, the rubbing number does not reach 100 times at 120°C. On the other hand, in view of the fact that the rubbing number reaches 100 times at 90°C in Examples 1, 2, 4 to 8, and 10 to 21 and at 100°C in Examples 3 and 9, respectively, it is understood that the quaternary ammonium salt has more excellent low-temperature blocking agent dissociation activity than dibutyltin dilaurate.

### Comparative Example 3

A nonaqueous thermosetting composition containing triethylmonomethylammonium 2-ethylhexanoate (U-CAT18X, manufactured by San-Apro Ltd.) as described in Patent Document 1 in a formulation shown in Table 5 was obtained in the same method as that in Comparative Example 2.

The obtained nonaqueous thermosetting composition was baked at 80°C, 90°C, 100°C, 110°C, and 120°C, respectively and then measured for the solvent resistance. The results are collectively shown in Table 5.

As is clear from Tables 1 to 5, in Comparative Example 3, the rubbing number reaches 100 times at 120°C. On the other hand, in view of the fact that the rubbing number reaches 100 times at 90°C in Examples 1, 2, 4 to 8, and 10 to 21 and at 100°C in Examples 3 and 9, respectively, it is understood that the quaternary ammonium salt used in the present invention has more excellent low-temperature blocking agent dissociation activity than triethylmonomethylammonium 2-ethylhexanoate.

### Example 22 (Evaluation of catalytic activity of quaternary ammonium salt in aqueous thermosetting composition)

An aqueous acrylic polyol (WAP-768, manufactured by Asia Industry Co., Ltd., solid content concentration: 40.0% by weight, hydroxyl value relative to the solid content: 57.5 mgKOH/g) and the aqueous blocked isocyanate obtained in Production Example 2 were mixed in a formulation shown in Table 6, and thereafter, the 38.7% by weight ethylene glycol solution of trimethylmono-n-decylammonium hydrogencarbonate obtained in Production Example 6 was added thereto while stirring, and thereby an aqueous thermosetting composition containing trimethylmono-n-decylammonium hydrogencarbonate was obtained.

The obtained aqueous thermosetting composition was baked at 110°C, 120°C, 130°C, and 140°C, respectively and then measured for the solvent resistance. The results are collectively shown in Table 6.

**Table 6**

| | Example 22 | Example 23 | Example 24 | Comparative Example 4 |
|---|---|---|---|---|
| Aqueous acrylic polyol (1) | 10.0 g | 10.0 g | 10.0 g | 10.0 g |
| Aqueous blocked isocyanate (2) | 3.44 g | 3.44 g | 3.44 g | 3.44 g |
| Catalyst solution | 38.7% by weight ethylene glycol solution of trimethylmono-n-dec ylammonium hydrogen carbonate obtained in Production Example 6 | 40.0% by weight ethylene glycol solution of trimethylmono-n-dode cylammonium monomethylcarbonate obtained in Production Example 9 | 40.0% by weight ethylene glycol solution of trimethylmono-n-tet radecylammonium carbonate obtained in Production Example 17 | No |
| | 0.138 g | 0.133 g | 0.133 g | |
| Proportion of catalyst to solid content | 1 % by weight | 1% by weight | 1 % by weight | 0% by weight |
| Solvent resistance (rubbing number) | | | | |
| 110°C ⁽³⁾ | 10 | 15 | 10 | <5 |
| 120°C ⁽³⁾ | >100 | >100 | >100 | <5 |
| 130°C ⁽³⁾ | >100 | >100 | >100 | 50 |
| 140°C ⁽³⁾ | >100 | >100 | >100 | >100 |
| (1) WAP-768, manufactured by Asia Industry Co., Ltd. | | | | |
| (2) Aqueous blocked isocyanate obtained in Production Example 2 | | | | |
| (3) Baking temperature | | | | |

### Examples 23 to 24

Aqueous thermosetting compositions containing a quaternary ammonium salt were obtained in formations shown in Table 6 by the same method as that in Example 22.

Each of the obtained aqueous thermosetting compositions was baked at 110°C, 120°C, 130°C, and 140°C, respectively and then measured for the solvent resistance. The results are collectively shown in Table 6.

### Comparative Example 4 (Evaluation of curability in non-addition of catalyst in aqueous thermosetting composition)

An aqueous acrylic polyol (WAP-768, manufactured by Asia Industry Co., Ltd., solid content concentration: 40.0% by weight, hydroxyl value relative to the solid content: 57.5 mgKOH/g) and the aqueous blocked isocyanate obtained in Production Example 2 were mixed in a formulation shown in Table 6, and thereby a catalyst-free aqueous thermosetting composition was obtained.

The obtained aqueous thermosetting composition was baked at 110°C, 120°C, 130°C, and 140°C, respectively and then measured for the solvent resistance. The results are shown in Table 6.

As is clear from Table 6, in Comparative Example 4, the rubbing number reaches 100 times at 140°C. On the other hand, in view of the fact that the rubbing number reaches 100 times at 120°C in Examples 22 to 24, it is understood that the dissociation temperature of the blocking agent was lowered by the addition of the quaternary ammonium salt.

### Industrial Applicability

The catalyst for dissociation of a blocking agent of the present disclosure is utilized as a catalyst for dissociating a blocking agent of a polyisocyanate, and the thermosetting composition containing the catalyst for dissociation of a blocking agent, a blocked isocyanate, and a compound having an isocyanate reactive group is baked and used as a thermosetting resin having excellent solvent resistance.

## Claims

1. A thermosetting composition, which comprises:
a catalyst for dissociation of a blocking agent, said catalyst for dissociation of a blocking agent comprising: a quaternary ammonium salt represented by the following general formula (1): 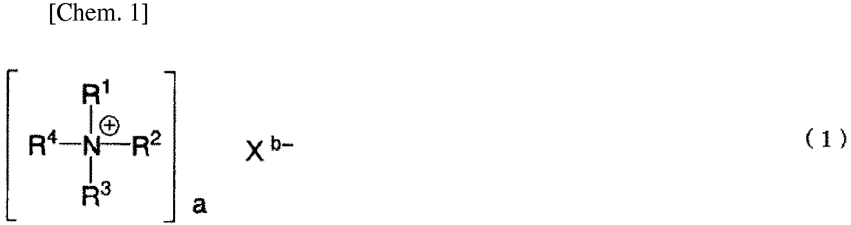 wherein each of R¹ to R³ represents an aliphatic hydrocarbon group having 1 to 8 carbon atoms or an aromatic hydrocarbon group having 6 to 14 carbon atoms; R⁴ represents an aliphatic hydrocarbon group having 9 to 20 carbon atoms; X represents at least one member selected from the group consisting of a phosphate group, a borate group, a hydrogencarbonate group, a monoalkylcarbonate group and a carbonate group; a is an integer in the range of 1 to 3; and b is an integer in the range of 1 to 3;
a blocked isocyanate; and
a compound having an isocyanate reactive group.

2. The thermosetting composition according to claim 1,
wherein the quaternary ammonium salt is at least one quaternary ammonium salt selected from the group consisting of trimethylmono-n-decylammonium orthophosphate, trimethylmono-n-decylammonium orthoborate, trimethylmono-n-decylammonium hydrogencarbonate, trimethylmono-n-decylammonium monomethylcarbonate, dimethylmonoethylmono-n-decylammonium monoethylcarbonate, trimethylmono-n-decylammonium carbonate, trimethylmono-n-dodecylammonium orthophosphate, trimethylmono-n-dodecylammonium orthoborate, trimethylmono-n-dodecylammonium hydrogencarbonate, trimethylmono-n-dodecylammonium monomethylcarbonate, dimethylmonoethylmono-n-dodecylammonium monoethylcarbonate, trimethylmono-n-dodecylammonium carbonate, trimethylmono-n-tetradecylammonium hydrogencarbonate, trimethylmono-n-tetradecylammonium monomethylcarbonate, trimethylmono-n-tetradecylammonium carbonate, trimethylmono-n-hexadecylammonium hydrogencarbonate, trimethylmono-n-hexadecylammonium monomethylcarbonate, trimethylmono-n-hexadecylammonium carbonate, trimethylmono-n-octadecylammonium hydrogencarbonate, trimethylmono-n-octadecylammonium monomethylcarbonate and trimethylmono-n-octadecylammonium carbonate.

3. The thermosetting composition according to claim 1 or 2,
wherein the compound having an isocyanate reactive group is a polyol.

4. Use of a catalyst for dissociation of a blocking agent, wherein said catalyst for dissociation of a blocking agent comprises: a quaternary ammonium salt represented by the following general formula (1): 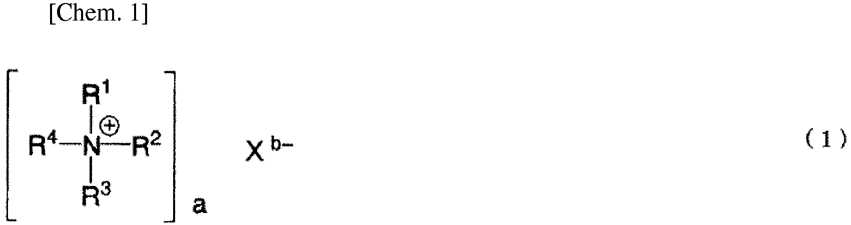 wherein each of R¹ to R³ represents an aliphatic hydrocarbon group having 1 to 8 carbon atoms or an aromatic hydrocarbon group having 6 to 14 carbon atoms; R⁴ represents an aliphatic hydrocarbon group having 9 to 20 carbon atoms; X represents at least one member selected from the group consisting of a phosphate group, a borate group, a hydrogencarbonate group, a monoalkylcarbonate group and a carbonate group; a is an integer in the range of 1 to 3; and b is an integer in the range of 1 to 3,
for dissociating a blocking agent from a blocked isocyanate.

5. Use of the catalyst for dissociation of a blocking agent according to claim 4,
wherein the quaternary ammonium salt is at least one quaternary ammonium salt selected from the group consisting of trimethylmono-n-decylammonium orthophosphate, trimethylmono-n-decylammonium orthoborate, trimethylmono-n-decylammonium hydrogencarbonate, trimethylmono-n-decylammonium monomethylcarbonate, dimethylmonoethylmono-n-decylammonium monoethylcarbonate, trimethylmono-n-decylammonium carbonate, trimethylmono-n-dodecylammonium orthophosphate, trimethylmono-n-dodecylammonium orthoborate, trimethylmono-n-dodecylammonium hydrogencarbonate, trimethylmono-n-dodecylammonium monomethylcarbonate, dimethylmonoethylmono-n-dodecylammonium monoethylcarbonate, trimethylmono-n-dodecylammonium carbonate, trimethylmono-n-tetradecylammonium hydrogencarbonate, trimethylmono-n-tetradecylammonium monomethylcarbonate, trimethylmono-n-tetradecylammonium carbonate, trimethylmono-n-hexadecylammonium hydrogencarbonate, trimethylmono-n-hexadecylammonium monomethylcarbonate, trimethylmono-n-hexadecylammonium carbonate, trimethylmono-n-octadecylammonium hydrogencarbonate, trimethylmono-n-octadecylammonium monomethylcarbonate and trimethylmono-n-octadecylammonium carbonate.

6. A method for producing a thermosetting composition, which comprises:
reacting a blocked isocyanate and a compound having an isocyanate reactive group,
in the presence of a catalyst for dissociation of a blocking agent, said catalyst for dissociation of a blocking agent comprising: a quaternary ammonium salt represented by the following general formula (1): 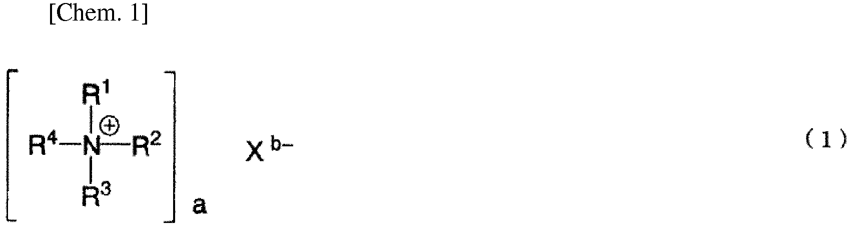 wherein each of R¹ to R³ represents an aliphatic hydrocarbon group having 1 to 8 carbon atoms or an aromatic hydrocarbon group having 6 to 14 carbon atoms; R⁴ represents an aliphatic hydrocarbon group having 9 to 20 carbon atoms; X represents at least one member selected from the group consisting of a phosphate group, a borate group, a hydrogencarbonate group, a monoalkylcarbonate group and a carbonate group; a is an integer in the range of 1 to 3; and b is an integer in the range of 1 to 3,
wherein the thermosetting composition comprises:
the catalyst for dissociation of a blocking agent as defined above;
a blocked isocyanate; and
a compound having an isocyanate reactive group.

7. The method for producing a thermosetting composition according to claim 6,
wherein the quaternary ammonium salt is at least one quaternary ammonium salt selected from the group consisting of trimethylmono-n-decylammonium orthophosphate, trimethylmono-n-decylammonium orthoborate, trimethylmono-n-decylammonium hydrogencarbonate, trimethylmono-n-decylammonium monomethylcarbonate, dimethylmonoethylmono-n-decylammonium monoethylcarbonate, trimethylmono-n-decylammonium carbonate, trimethylmono-n-dodecylammonium orthophosphate, trimethylmono-n-dodecylammonium orthoborate, trimethylmono-n-dodecylammonium hydrogencarbonate, trimethylmono-n-dodecylammonium monomethylcarbonate, dimethylmonoethylmono-n-dodecylammonium monoethylcarbonate, trimethylmono-n-dodecylammonium carbonate, trimethylmono-n-tetradecylammonium hydrogencarbonate, trimethylmono-n-tetradecylammonium monomethylcarbonate, trimethylmono-n-tetradecylammonium carbonate, trimethylmono-n-hexadecylammonium hydrogencarbonate, trimethylmono-n-hexadecylammonium monomethylcarbonate, trimethylmono-n-hexadecylammonium carbonate, trimethylmono-n-octadecylammonium hydrogencarbonate, trimethylmono-n-octadecylammonium monomethylcarbonate and trimethylmono-n-octadecylammonium carbonate.

## Patentansprüche

1. Wärmehärtende Zusammensetzung umfassend:
einen Katalysator zur Dissoziation eines Blockierungsmittels, wobei der Katalysator zur Dissoziation eines Blockierungsmittels Folgendes umfasst: ein quaternäres Ammoniumsalz, das von nachfolgender allgemeiner Formel (1) dargestellt wird: 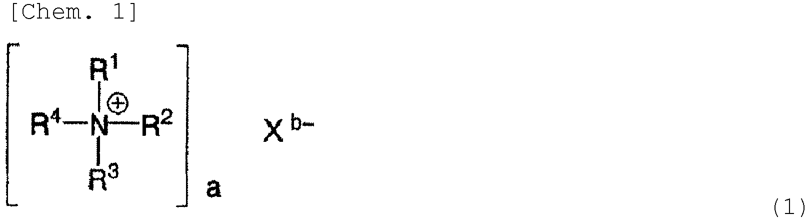 wobei jedes von R¹ bis R³ eine aliphatische Kohlenwasserstoffgruppe mit 1 bis 8 Kohlenstoffatomen oder eine aromatische Kohlenwasserstoffgruppe mit 6 bis 14 Kohlenstoffatomen darstellt; R⁴ eine aliphatische Kohlenwasserstoffgruppe mit 9 bis 20 Kohlenstoffatomen darstellt; X zumindest ein Element darstellt, das aus der Gruppe bestehend aus einer Phosphatgruppe, einer Boratgruppe, einer Wasserstoffcarbonatgruppe, einer Monoalkylcarbonatgruppe und einer Carbonatgruppe ausgewählt wird; a eine ganze Zahl im Bereich von 1 bis 3 ist; und b eine ganze Zahl im Bereich von 1 bis 3 ist;
ein blockiertes Isocyanat; und
eine Verbindung mit einer Isocyanat-reaktiven Gruppe.

2. Wärmehärtende Zusammensetzung nach Anspruch 1,
wobei das quaternäre Ammoniumsalz zumindest ein quaternäres Ammoniumsalz ist, das aus der Gruppe bestehend aus Trimethylmono-n-decylammonium-Orthophosphat, Trimethylmono-n-decylammonium-Orthoborat, Trimethylmono-n-decylammonium-Wasserstoffcarbonat, Trimethylmono-n-decylammonium-Monomethylcarbonat, Dimethylmonoethylmono-n-decylammonium-Monoethylcarbonat, Trimethylmono-n-decylammoniumcarbonat, Trimethylmono-n-dodecylammonium-Orthophosphat, Trimethylmono-n-dodecylammonium-Orthoborat, Trimethylmono-n-dodecylammonium-Wasserstoffcarbonat, Trimethylmono-n-dodecylammonium-Monomethylcarbonat, Dimethylmonoethylmono-n-dodecylammonium-Monoethylcarbonat, Trimethylmono-n-dodecylammoniumcarbonat, Trimethylmono-n-tetradecylammonium-Wasserstoffcarbonat, Trimethylmono-n-tetradecylammonium-Monomethylcarbonat, Trimethylmono-n-tetradecylammoniumcarbonat, Trimethylmono-n-hexadecylammonium-Wasserstoffcarbonat, Trimethylmono-n-hexadecylammonium-Monomethylcarbonat, Trimethylmono-n-hexadecylammoniumcarbonat, Trimethylmono-n-octadecylammonium-Wasserstoffcarbonat, Trimethylmono-n-octadecylammoniumMonomethylcarbonat und Trimethylmono-n-octadecylammoniumcarbonat ausgewählt ist.

3. Wärmehärtende Zusammensetzung nach Anspruch 1 oder 2, wobei die Verbindung mit einer mit Isocyanat-reaktiven Gruppe ein Polyol ist.

4. Verwendung eines Katalysators zur Dissoziation eines Blockierungsmittels, wobei der Katalysator zur Dissoziation eines Blockierungsmittels Folgendes umfasst: ein quaternäres Ammoniumsalz, dargestellt durch die folgende allgemeine Formel (1) : 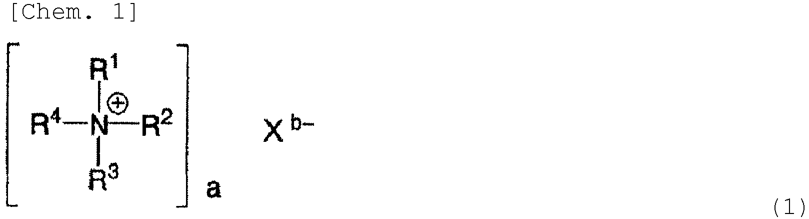
wobei jedes von R¹ bis R³ eine aliphatische Kohlenwasserstoffgruppe mit 1 bis 8 Kohlenstoffatomen oder eine aromatische Kohlenwasserstoffgruppe mit 6 bis 14 Kohlenstoffatomen darstellt; R⁴ eine aliphatische Kohlenwasserstoffgruppe mit 9 bis 20 Kohlenstoffatomen darstellt; X zumindest ein Element darstellt, das aus der Gruppe bestehend aus einer Phosphatgruppe, einer Boratgruppe, einer Wasserstoffcarbonatgruppe, einer Monoalkylcarbonatgruppe und einer Carbonatgruppe ausgewählt ist; a eine ganze Zahl im Bereich von 1 bis 3 ist; und b eine ganze Zahl im Bereich von 1 bis 3 ist,
zur Dissoziation eines Blockierungsmittels von einem blockierten Isocyanat.

5. Verwendung des Katalysators zur Dissoziation eines Blockierungsmittels nach Anspruch 4,
wobei das quaternäre Ammoniumsalz zumindest ein quaternäres Ammoniumsalz ist, das aus der Gruppe bestehend aus Trimethylmono-n-decylammonium-Orthophosphat, Trimethylmono-n-decylammonium-Orthoborat, Trimethylmono-n-decylammonium-Wasserstoffcarbonat, Trimethylmono-n-decylammonium-Monomethylcarbonat, Dimethylmonoethylmono-n-decylammonium-Monoethylcarbonat, Trimethylmono-n-decylammonium-Carbonat, Trimethylmono-n-dodecylammonium-Orthophosphat, Trimethylmono-n-dodecylammonium-Orthoborat, Trimethylmono-n-dodecylammonium-Wasserstoffcarbonat, Trimethylmono-n-dodecylammoniummonomethylcarbonat, Dimethylmonoethylmono-n-dodecylammonium-Monoethylcarbonat, Trimethylmono-n-dodecylammoniumcarbonat, Trimethylmono-n-tetradecylammonium-Wasserstoffcarbonat, Trimethylmono-n-tetradecylammonium-Monomethylcarbonat, Trimethylmono-n-tetradecylammoniumcarbonat, Trimethylmono-n-hexadecylammonium-Wasserstoffcarbonat, Trimethylmono-n-hexadecylammonium-Monomethylcarbonat, Trimethylmono-n-hexadecylammoniumcarbonat, Trimethylmono-n-octadecylammonium-Wasserstoffcarbonat, Trimethylmono-n-octadecylammoniummonomethylcarbonat und Trimethylmono-n-octadecylammoniumcarbonat ausgewählt ist.

6. Verfahren zur Herstellung einer wärmehärtenden Zusammensetzung umfassend:
Reaktion eines blockierten Isocyanats und einer Verbindung mit einer Isocyanat-reaktiven Gruppe,
in Gegenwart eines Katalysators zur Dissoziation eines Blockierungsmittels, wobei der Katalysator zur Dissoziation eines Blockierungsmittels Folgendes umfasst: ein quaternäres Ammoniumsalz, das durch die folgende allgemeine Formel (1) dargestellt wird: 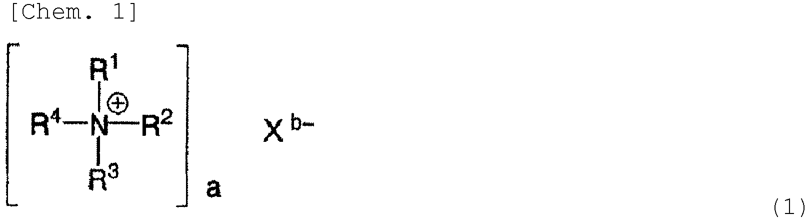 wobei jedes von R¹ bis R³ eine aliphatische Kohlenwasserstoffgruppe mit 1 bis 8 Kohlenstoffatomen oder eine aromatische Kohlenwasserstoffgruppe mit 6 bis 14 Kohlenstoffatomen darstellt; R⁴ eine aliphatische Kohlenwasserstoffgruppe mit 9 bis 20 Kohlenstoffatomen darstellt; X zumindest ein Element darstellt, das aus der Gruppe bestehend aus einer Phosphatgruppe, einer Boratgruppe, einer Wasserstoffcarbonatgruppe, einer Monoalkylcarbonatgruppe und einer Carbonatgruppe ausgewählt ist; a eine ganze Zahl im Bereich von 1 bis 3 ist; und b eine ganze Zahl im Bereich von 1 bis 3 ist,
wobei die wärmehärtende Zusammensetzung Folgendes umfasst:
den Katalysator zur Dissoziation eines Blockierungsmittels wie oben definiert;
ein blockiertes Isocyanat; und
eine Verbindung mit einer Isocyanat-reaktiven Gruppe.

7. Verfahren zur Herstellung einer wärmehärtenden Zusammensetzung nach Anspruch 6,
wobei das quaternäre Ammoniumsalz zumindest ein quaternäres Ammoniumsalz ist, das aus der Gruppe bestehend aus Trimethylmono-n-decylammonium-Orthophosphat, Trimethylmono-n-decylammonium-Orthoborat, Trimethylmono-n-decylammonium-Wasserstoffcarbonat, Trimethylmono-n-decylammonium-Monomethylcarbonat, Dimethylmonoethylmono-n-decylammonium-Monoethylcarbonat, Trimethylmono-n-decylammoniumcarbonat, Trimethylmono-n-dodecylammonium-Orthophosphat, Trimethylmono-n-dodecylammonium-Orthoborat, Trimethylmono-n-dodecylammonium-Wasserstoffcarbonat, Trimethylmono-n-dodecylammonium-Monomethylcarbonat, Dimethylmonoethylmono-n-dodecylammonium-Monoethylcarbonat, Trimethylmono-n-dodecylammoniumcarbonat, Trimethylmono-n-tetradecylammonium-Wasserstoffcarbonat, Trimethylmono-n-tetradecylammonium-Monomethylcarbonat, Trimethylmono-n-tetradecylammoniumcarbonat, Trimethylmono-n-hexadecylammonium-Wasserstoffcarbonat, Trimethylmono-n-hexadecylammonium-Monomethylcarbonat, Trimethylmono-n-hexadecylammoniumcarbonat, Trimethylmono-n-octadecylammonium-Wasserstoffcarbonat, Trimethylmono-n-octadecylammoniumMonomethylcarbonat und Trimethylmono-n-octadecylammoniumcarbonat ausgewählt ist.

## Revendications

1. Composition thermodurcissable, qui comprend :
un catalyseur pour la dissociation d'un agent de blocage, ledit catalyseur pour la dissociation d'un agent de blocage comprenant : un sel d'ammonium quaternaire représenté par la formule générale (1) suivante : 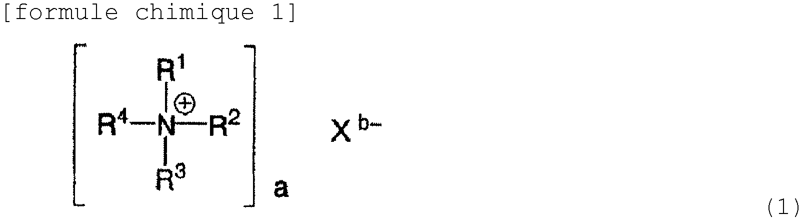 dans laquelle chacun de R¹ à R³ représente un groupe hydrocarboné aliphatique ayant 1 à 8 atomes de carbone ou un groupe hydrocarboné aromatique ayant 6 à 14 atomes de carbone ; R⁴ représente un groupe hydrocarboné aliphatique ayant 9 à 20 atomes de carbone ; X représente au moins un membre choisi dans l'ensemble constitué par un groupe phosphate, un groupe borate, un groupe hydrogénocarbonate, un groupe monoalkylcarbonate et un groupe carbonate ; a est un entier situé dans la plage allant de 1 à 3 ; et b est un entier situé dans la plage allant de 1 à 3 ;
un isocyanate bloqué ; et
un composé ayant un groupe réactif vis-à-vis des isocyanates.

2. Composition thermodurcissable selon la revendication 1, dans lequel le sel d'ammonium quaternaire est au moins un sel d'ammonium quaternaire choisi dans l'ensemble constitué par l'orthophosphate de triméthylmono-n-décylammonium, l'orthoborate de triméthylmono-n-décylammonium, l'hydrogénocarbonate de triméthylmono-n-décylammonium, le monométhylcarbonate de triméthylmono-n-décylammonium, le monoéthylcarbonate de diméthylmonoéthylmono-n-décylammonium, le carbonate de triméthylmono-n-décylammonium, l'orthophosphate de triméthylmono-n-dodécylammonium, l'orthoborate de triméthylmono-n-dodécylammonium, l'hydrogénocarbonate de triméthylmono-n-dodécylammonium, le monométhylcarbonate de triméthylmono-n-dodécylammonium, le monoéthylcarbonate de diméthylmonoéthylmono-n-dodécylammonium, le carbonate de triméthylmono-n-dodécylammonium, l'hydrogénocarbonate de triméthylmono-n-tétradécylammonium, le monométhylcarbonate de triméthylmono-n-tétradécylammonium, le carbonate de triméthylmono-n-tétradécylammonium, l'hydrogénocarbonate de triméthylmono-n-hexadécylammonium, le monométhylcarbonate de triméthylmono-n-hexadécylammonium, le carbonate de triméthylmono-n-hexadécylammonium, l'hydrogénocarbonate de triméthylmono-n-octadécylammonium, le monométhylcarbonate de triméthylmono-n-octadécylammonium, et le carbonate de triméthylmono-n-octadécylammonium.

3. Composition thermodurcissable selon la revendication 1 ou 2,
dans laquelle le composé ayant un groupe réactif vis-à-vis des isocyanates est un polyol.

4. Utilisation d'un catalyseur pour la dissociation d'un agent de blocage, dans laquelle ledit catalyseur pour la dissociation d'un agent de blocage comprend : un sel d'ammonium quaternaire représenté par la formule générale (1) suivante : 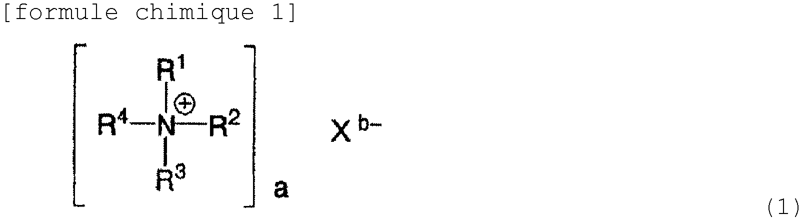
dans laquelle chacun de R¹ à R³ représente un groupe hydrocarboné aliphatique ayant 1 à 8 atomes de carbone ou un groupe hydrocarboné aromatique ayant 6 à 14 atomes de carbone ; R⁴ représente un groupe hydrocarboné aliphatique ayant 9 à 20 atomes de carbone ; X représente au moins un membre choisi dans l'ensemble constitué par un groupe phosphate, un groupe borate, un groupe hydrogénocarbonate, un groupe monoalkylcarbonate et un groupe carbonate ; a est un entier situé dans la plage allant de 1 à 3 ; et b est un entier situé dans la plage allant de 1 à 3,
pour dissocier un agent de blocage d'un isocyanate bloqué.

5. Utilisation d'un catalyseur pour la dissociation d'un agent de blocage selon la revendication 4,
dans laquelle le sel d'ammonium quaternaire est au moins un sel d'ammonium quaternaire choisi dans l'ensemble constitué par l'orthophosphate de triméthylmono-n-décylammonium, l'orthoborate de triméthylmono-n-décylammonium, l'hydrogénocarbonate de triméthylmono-n-décylammonium, le monométhylcarbonate de triméthylmono-n-décylammonium, le monoéthylcarbonate de diméthylmonoéthylmono-n-décylammonium, le carbonate de triméthylmono-n-décylammonium, l'orthophosphate de triméthylmono-n-dodécylammonium, l'orthoborate de triméthylmono-n-dodécylammonium, l'hydrogénocarbonate de triméthylmono-n-dodécylammonium, le monométhylcarbonate de triméthylmono-n-dodécylammonium, le monoéthylcarbonate de diméthylmonoéthylmono-n-dodécylammonium, le carbonate de triméthylmono-n-dodécylammonium, l'hydrogénocarbonate de triméthylmono-n-tétradécylammonium, le monométhylcarbonate de triméthylmono-n-tétradécylammonium, le carbonate de triméthylmono-n-tétradécylammonium, l'hydrogénocarbonate de triméthylmono-n-hexadécylammonium, le monométhylcarbonate de triméthylmono-n-hexadécylammonium, le carbonate de triméthylmono-n-hexadécylammonium, l'hydrogénocarbonate de triméthylmono-n-octadécylammonium, le monométhylcarbonate de triméthylmono-n-octadécylammonium, et le carbonate de triméthylmono-n-octadécylammonium.

6. Procédé pour produire une composition thermodurcissable, qui comprend :
la réaction d'un isocyanate bloqué et d'un composé ayant un groupe réactif vis-à-vis des isocyanates,
en présence d'un catalyseur pour la dissociation d'un agent de blocage, ledit catalyseur pour la dissociation d'un agent de blocage comprenant :
un sel d'ammonium quaternaire représenté par la formule générale (1) suivante : 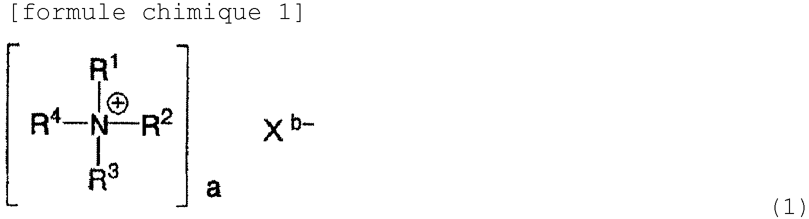 dans laquelle chacun de R¹ à R³ représente un groupe hydrocarboné aliphatique ayant 1 à 8 atomes de carbone ou un groupe hydrocarboné aromatique ayant 6 à 14 atomes de carbone ; R⁴ représente un groupe hydrocarboné aliphatique ayant 9 à 20 atomes de carbone ; X représente au moins un membre choisi dans l'ensemble constitué par un groupe phosphate, un groupe borate, un groupe hydrogénocarbonate, un groupe monoalkylcarbonate et un groupe carbonate ; a est un entier situé dans la plage allant de 1 à 3 ; et b est un entier situé dans la plage allant de 1 à 3,
dans lequel la composition thermodurcissable comprend :
le catalyseur pour la dissociation d'un agent de blocage tel que défini ci-dessus ;
un isocyanate bloqué ; et
un composé ayant un groupe réactif vis-à-vis des isocyanates.

7. Procédé pour produire une composition thermodurcissable selon la revendication 6,
dans lequel le sel d'ammonium quaternaire est au moins un sel d'ammonium quaternaire choisi dans l'ensemble constitué par l'orthophosphate de triméthylmono-n-décylammonium, l'orthoborate de triméthylmono-n-décylammonium, l'hydrogénocarbonate de triméthylmono-n-décylammonium, le monométhylcarbonate de triméthylmono-n-décylammonium, le monoéthylcarbonate de diméthylmonoéthylmono-n-décylammonium, le carbonate de triméthylmono-n-décylammonium, l'orthophosphate de triméthylmono-n-dodécylammonium, l'orthoborate de triméthylmono-n-dodécylammonium, l'hydrogénocarbonate de triméthylmono-n-dodécylammonium, le monométhylcarbonate de triméthylmono-n-dodécylammonium, le monoéthylcarbonate de diméthylmonoéthylmono-n-dodécylammonium, le carbonate de triméthylmono-n-dodécylammonium, l'hydrogénocarbonate de triméthylmono-n-tétradécylammonium, le monométhylcarbonate de triméthylmono-n-tétradécylammonium, le carbonate de triméthylmono-n-tétradécylammonium, l'hydrogénocarbonate de triméthylmono-n-hexadécylammonium, le monométhylcarbonate de triméthylmono-n-hexadécylammonium, le carbonate de triméthylmono-n-hexadécylammonium, l'hydrogénocarbonate de triméthylmono-n-octadécylammonium, le monométhylcarbonate de triméthylmono-n-octadécylammonium, et le carbonate de triméthylmono-n-octadécylammonium.
